# EUROPÄISCHE PATENTSCHRIFT

(11) **EP 1 685 381 B1**
(45) Veröffentlichungstag und Bekanntmachung des Hinweises auf die Patenterteilung: **21.03.2007**
(21) Anmeldenummer: 04818791.8
(22) Anmeldetag: 17.11.2004
(51) Int. Cl.: G01N 1/00, G01N 1/40

(54) **VORRICHTUNG ZUM ABTRENNEN FLÜCHTIGER ORGANISCHER KOHLENSTOFFVERBINDUNGEN VON EINER PROZESSFLÜSSIGKEIT**
DEVICE FOR REMOVING VOLATILE ORGANIC CARBON COMPOUNDS FROM A PROCESS LIQUID
DISPOSITIF POUR SEPARER DES COMPOSES CARBONE ORGANIQUES VOLATILS CONTENUS DANS UN LIQUIDE DE TRAITEMENT

(30) Priorität: 17.11.2003 DE 20317754 U
(43) Veröffentlichungstag der Anmeldung: 02.08.2006
(73) Patentinhaber: TH Techno Holding GmbH, 30989 Gehrden (DE)
(72) Erfinder: BERNATH, Tibor, 30989 Gehrden (DE)
(74) Vertreter: Walcher, Armin
(86) Internationale Anmeldenummer: PCT/EP2004/013023
(87) Internationale Veröffentlichungsnummer: WO 2005/050173

(56) Entgegenhaltungen:
- DE-A1- 2 713 621
- DE-A1- 2 810 292
- DE-A1- 4 243 121
- DE-U1- 8 804 409
- MURATA A; SHIBATA S; SAITO M: "Continuous Analyzer for Volatile Organic Compounds in Air and Water" YOKOGAWA TECHNICAL REPORT ENGLISH EDITION, Nr. 31, 2001, Seiten 1-4, XP002325447 JAPAN in der Anmeldung erwähnt

## Beschreibung

Die Erfindung betrifft eine Vorrichtung zum Abtrennen flüchtiger organischer Kohlenstoffverbindungen (VOC = Volatile Organic Carbon) von einer Prozessflüssigkeit, insbesondere von Wasser, gemäß dem Oberbegriff des Anspruchs 1.

Kohlenstoffverbindungen sind in zwei Gruppen aufgeteilt, in anorganische und in organische Substanzen. Im Gegensatz zu den anorganischen Kohlenstoffverbindungen sind Tausende von Verbindungen organischer Natur bekannt. Je nach Substanz sind diese sowohl für den Menschen als auch für die Umwelt schädlich. Aus diesem Grunde ist die Messung und Überwachung der organisch gebundenen Kohlenstoffe essentiell.

Die Bestimmung kann für eine Einzelsubstanz oder aber als Kohlenstoffsumme erfolgen. Für die Überwachung von Flüssigkeiten wie Industrieabwässer oder Kühlkreisläufe erfolgt die Bestimmung in zwei Stufen: eine aufwändige und zeitintensive Einzelsubstanzanalyse wird erst dann durchgeführt, wenn eine vorher festgelegte Grenze der Summe des organisch gebundenen Kohlenstoffs überschritten ist. Aus diesem Grunde sind schnell und einfach arbeitende Messgeräte für die Gesamtkohlenstoffmessung, auch Total Organic Carbon (TOC) genannt, erforderlich.

Aus der Veröffentlichung Yokogawa Technical Report English Edition, No. 31 (2001), Seite 1 bis 4, der Firma Yokogawa Electric Corporation ist ein Analysator zur Gesamtkohlenstoffmessung bekannt, mit dem flüchtige organische Kohlenstoffverbindungen (VOC) in Wasser gemessen werden. Dazu werden die VOC-Komponenten mittels einer gattungsgemäßen Vorrichtung von dem Wasser abgetrennt. Die Trennvorrichtung besteht aus einem Behältnis, in dem sich das Prozesswasser befindet und das kontinuierlich mit sauberer Luft als Messgas durchströmt wird. Dabei wird das Messgas mit den flüchtigen Bestandteilen des organisch gebundenen Kohlenstoffs gesättigt. Vorrichtungen dieser Art werden auch als Strippeinrichtung oder Stripper bezeichnet. Da das Messgas nicht nur die VOC-Komponenten aufnimmt, sondern auch mit Wasser selbst gesättigt wird, wird das Messgas vor der Analyse einem Entfeuchter zugeführt. Danach wird das Messgas mittels einer externen Pumpe einem Gesamtkohlenstoff-Gasanalysator zugeführt, mit dem die Messung der VOC-Komponenten erfolgt. Die Gasanalysatoren können unterschiedliche VOC-sensitive Detektoren wie z. B. Flammenionisationsdetektoren, Photoionisationsdetektoren, Halbleiterdetektoren oder sonstige Detektoren beinhalten. Anstelle des Entfeuchters könnten auch die Leitungen, die das gesättigte Messgas führen, heizbar ausgeführt werden.

Ausgehend von diesem Stand der Technik ist es Aufgabe der Erfindung, eine möglichst einfach arbeitende und entsprechend wartungsarme und kostengünstige Vorrichtung zum Abtrennen bereitzustellen, um eine schnelle und einfache Gesamtkohlenstoffmessung zu ermöglichen.

Diese Aufgabe wird gelöst durch eine Vorrichtung mit den Merkmalen des Anspruchs 1.

Die erfindungsgemäße Vorrichtung zum Abtrennen flüchtiger organischer Kohlenstoffverbindungen von einer Prozessflüssigkeit, insbesondere von Wasser, weist ein Trennbehältnis mit Prozessflüssigkeitszulauf, Messgaszuführung, Prozessflüssigkeitsablauf und Messgasabführung auf, wobei im Prozessflüssigkeitsablauf eine Staudruckeinrichtung vorgesehen ist, so dass in der Messgasabführung ein konstanter Druck aufrechterhaltbar ist.

Ähnlich aufgebaute Vorrichtungen sind z.B. aus DE 2 713 621 und DE 2 810 292 bekannt, in diesen herrscht aber nur Atmosphärendruck.

Die Staudruckeinrichtung stellt sicher, dass im Prozessflüssigkeitsablauf stets ein konstant hoher Druck herrscht, so dass das Messgas ohne eine externe Pumpe zu einem nachgeschalteten Analysegerät befördert werden kann. Durch Verzicht auf die externe Pumpe können nicht nur Kosten eingespart werden, sondern ist der Aufbau auch wesentlich vereinfacht und entsprechend wartungsärmer.

Als vorteilhaft hat sich ein Staudruck von etwa 4 kPa erwiesen.

In konstruktiv einfacher und damit vorteilhafterweise ist die Staudruckeinrichtung als Staudruckgefäß ausgebildet.

Um eine reproduzierbare und langzeitstabile Phasenwandlung zu erreichen, wird neben dem Staudruck auch die Temperatur der Prozessflüssigkeit konstant gehalten, wozu im Prozessflüssigkeitszulauf eine Vorwärmeinheit vorgesehen ist. Darüber hinaus kann auch der Luftdurchsatz mittels eines Druckreglers und einer Drossel konstant gehalten werden.

Bevorzugt wird als Messgas Luft eingesetzt.

Damit in der Messgasleitung zwischen der erfindungsgemäßen Vorrichtung und einem nachgeschalteten Analysator keine störende Kondensation auftritt, ist in der Messgasabführung eine Kühleinrichtung zur Abkühlung des Messgases angeordnet, in der die von dem Messgas in dem Trennbehältnis aufgenommene Prozessflüssigkeit auskondensieren kann. Eine ansonsten zusätzliche Heizung zur Erwärmung der Leitung und zum Verhindern einer Kondensation in der Leitung kann entfallen.

Damit das Kondensat nicht weiter stören kann, ist die Kühlvorrichtung oberhalb des Trennbehältnisses und/oder des Staudruckgefäßes angeordnet, so dass in der Kühleinrichtung entstehende Kondensflüssigkeit in die Prozessflüssigkeit fließt und zusammen mit dieser abfließt. In wartungsfreier Weise entleert sich somit die Kühlvorrichtung kontinuierlich. Dazu ist vorteilhafterweise vorgesehen, dass die Kühleinrichtung eine untenliegende Zuleitung für das Messgas und eine obenliegende Ableitung aufweist, so dass die Kondensflüssigkeit durch die Messgaszuleitung wieder zurückfließen kann.

Vorteilhafterweise ist die Kühleinrichtung als Peltier-Kühler ausgebildet.

Eine Abkühlung des Messgases in der Kühleinrichtung auf etwa 2°C ist vorteilhaft, da der Taupunkt der flüchtigen organischen Stoffe niedriger als 2°C liegt und deshalb diese Stoffe in der Gasphase bleiben, wohingegen die Prozessflüssigkeit, wie Wasser, auskondensiert.

Die Erfindung wird im Folgenden anhand eines Ausführungsbeispiels unter Bezugnahme auf die Zeichnung im Einzelnen erläutert. In der Zeichnung zeigen:
- Fig. 1: ein Blockschaltbild einer erfindungsgemäßen Vorrichtung;
- Fig. 2: eine schematische Darstellung eines Messgaskühlers der Vorrichtung nach Fig. 1 entlang eines Schnittes II-II aus Fig. 3 gesehen;
- Fig. 3: eine schematische Darstellung des Messgaskühlers entlang eines Schnittes III-III aus Fig. 2 gesehen.

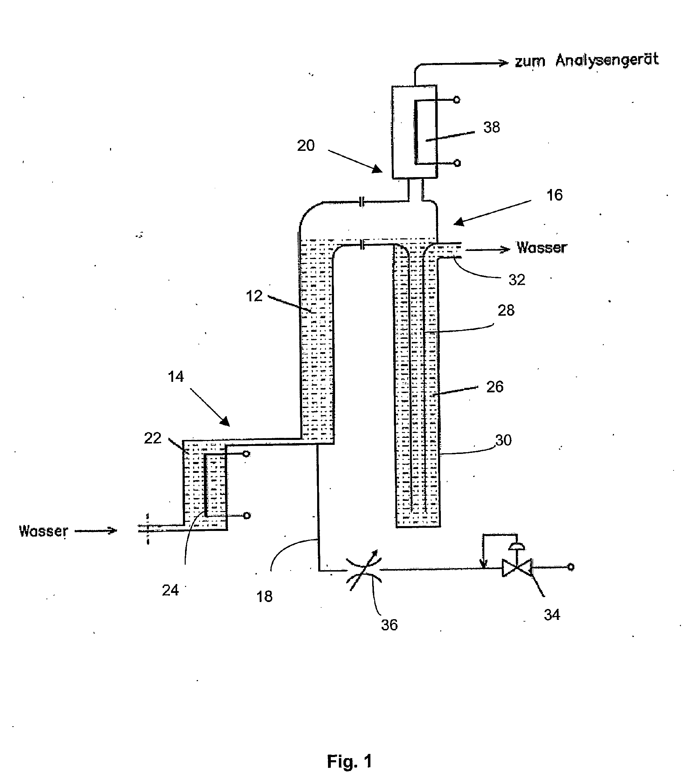

Eine in Fig. 1 dargestellte erfindungsgemäße Vorrichtung 10 zum Abtrennen flüchtiger organischer Kohlenstoffverbindungen (im Folgenden VOC genannt) von einer Prozessflüssigkeit, die in dem hier beschriebenen Ausführungsbeispiel Wasser ist, weist ein Trennbehältnis 12 auf, durch das einerseits das Wasser kontinuierlich strömt und andererseits gleichzeitig ein Messgas durch das Wasser strömt. Als Messgas wird in diesem Ausführungsbeispiel Luft eingesetzt.

Das Wasser fließt über einen Wasserzulauf 14 in das Trennbehältnis 12 ein und über einen Wasserablauf 16 aus dem Trennbehältnis 12 aus, wobei der Zulauf 14 in einem unteren Bereich und der Ablauf 16 in einem oberen Bereich angeordnet ist.

Über eine ebenfalls im unteren Bereich angeordneten Messgaszuführung 18 und eine in dem oberen Bereich angeordnete Messgasabführung 20 wird die Luft durch das Wasser geleitet, wobei die Luft als Luftblasen das Wasser von unten nach oben durchperlt und sich dabei mit den VOC-Komponenten und auch Wasserdampf anreichert.

Im Wasserzulauf ist eine Vorwärmeinheit 22 vorgesehen, in der mittels einer Heizung 24 das Wasser auf eine konstante Temperatur, die beispielsweise zwischen 40°C und 80°C liegen könnte, erwärmt wird. Eine konstante Temperatur fördert eine gewünschte Reproduzierbarkeit und Langzeitstabilität.

Im Wasserablauf ist eine als Staudruckgefäß 26 ausgebildete Staudruckeinrichtung angeordnet, die die Funktion hat, den Druck der gasförmigen Phase in dem Trennbehältnis 12 bzw. in der Messgasabführung 20 konstant zu halten, wobei ein Druck von 4 kPa bevorzugt ist. Aufgrund dieses konstanten Überdrucks in der Messgasabführung kann die Luft ohne eine zusätzliche externe Pumpe von der erfindungsgemäßen Vorrichtung 10 zu einem nicht dargestellten Analysegerät, in der der VOC-Gehalt des Messgases bestimmt wird, befördert werden. Das Staudruckgefäß 26 besteht in dem Ausführungsbeispiel in einfachster Weise aus einem Innen- 28 und Außenrohr 30, wobei die Höhe eines Wasserablasses 32 des Außenrohres 30 den Staudruck bestimmt.

Aus Gründen der Reproduzierbarkeit und Langzeitstabilität ist der Luftdurchsatz regelbar, indem in der Messgaszuführung 18 ein Druckregler 34 und eine Drossel 36 angeordnet sind.

In der Messgasabführung 20 ist eine Kühleinrichtung 38 angeordnet, die von dem Messgas durchströmt wird und in der das Messgas auf eine Temperatur abgekühlt wird, bei der das in dem Trennbehältnis 12 von der Luft unvermeidbar aufgenommene Wasser wieder auskondensiert.

Die Kühleinrichtung 38 ist oberhalb des Trennbehältnisses 12 und/oder des Staudruckgefässes 26 angeordnet, so dass die in der Kühleinrichtung 38 entstehende Kondensflüssigkeit in die Prozessflüssigkeit zurückfließt und zusammen mit dieser über den Ablauf 16 abfließt.

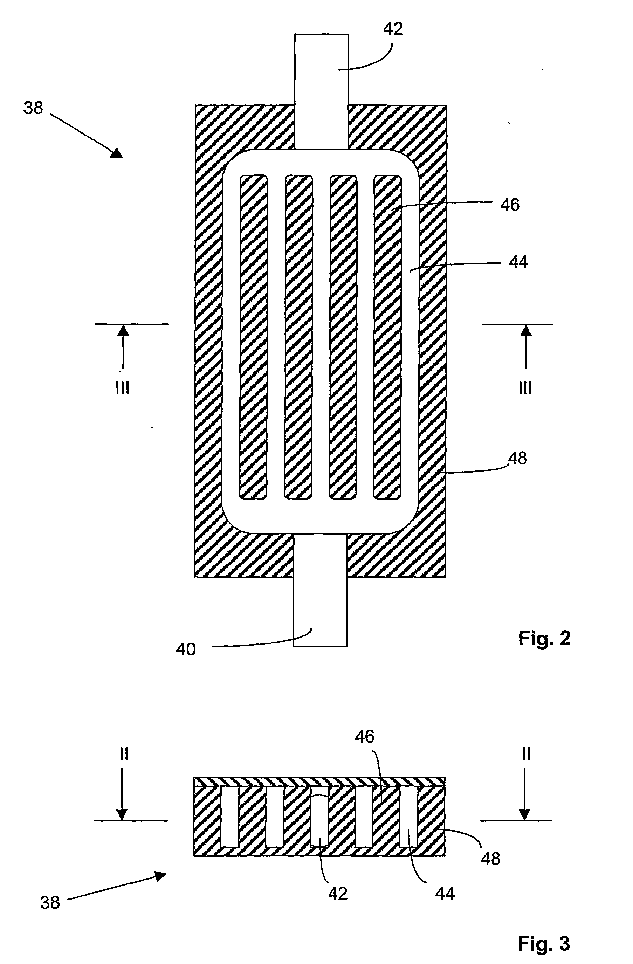

In den Fig. 2 und 3 ist eine mögliche Ausgestaltung einer solchen Kühleinrichtung 38 schematisch dargestellt. Die Kühleinrichtung 38 ist als Wärmetauscher ausgebildet und weist eine untenliegende Zuleitung 40 für das Messgas und eine obenliegende Ableitung 42 auf. Durch die untenliegende Messgaszuleitung 40 kann das Kondenswasser in das Trennbehältnis 12 oder das Staudruckgefäß 26 zurückfließen.

In einzelnen Kanälen 44 der Kühleinrichtung 38 strömt das Messgas und wird im Wärmetausch mit gekühlten Rippen 46 und Wandung 48 auf bevorzugt 2°C abgekühlt. Die Kühleinrichtung 38 ist bevorzugt als Peltier-Kühler ausgebildet. Damit kein Kondenswasser in der Kühleinrichtung verbleibt und möglichst alles Kondenswasser abfließen kann, sind die Kühlrippen 46 vertikal ausgerichtet. Gegebenenfalls könnten die Kühlrippen 46 auch eine aerodynamisch günstige Form aufweisen, damit kein allzu großer Druckabfall in der Kühleinrichtung 38 auftritt.

## Patentansprüche

1. Vorrichtung zum Abtrennen flüchtiger organischer Kohlenstoffverbindungen von einer Prozessflüssigkeit, insbesondere von Wasser, mit einem Trennbehältnis (12) mit Prozessflüssigkeitszulauf (14), Messgaszuführung (18), Prozessflüssigkeitsablauf (16) und Messgasabführung (20), wobei im Prozessflüssigkeitsablauf eine Staudruckeinrichtung (26) vorgesehen ist, so dass in der Messgasabführung (20) ein konstanter Druck aufrechterhaltbar ist, **dadurch gekennzeichnet, daß** die Vorrichtung so eingerichtet ist, daß die Gasphase in der Messgasabführung (20) einen Überdruck hat, der von der Staudruckeinrichtung Konstant gehalten wird.

2. Vorrichtung nach Anspruch 1, **dadurch gekennzeichnet, dass** die Staudruckeinrichtung als Staudruckgefäß (26) ausgebildet ist.

3. Vorrichtung nach einem der vorhergehenden Ansprüche, **dadurch gekennzeichnet, dass** der Staudruck etwa 4 kPa beträgt

4. Vorrichtung nach einem der vorhergehenden Ansprüche, **dadurch gekennzeichnet, dass** im Prozessflüssigkeitszulauf (14) eine Vorwärmeinheit (22) für die Prozessflüssigkeit vorgesehen ist.

5. Vorrichtung nach einem der vorhergehenden Ansprüche, **dadurch gekennzeichnet, dass** das Messgas Luft ist.

6. Vorrichtung nach einem der vorhergehenden Ansprüche, **dadurch gekennzeichnet, dass** in der Messgasabführung (20) eine Kühleinrichtung (38) zur Abkühlung des Messgases angeordnet ist.

7. Vorrichtung nach Anspruch 6, **dadurch gekennzeichnet, dass** die Kühleinrichtung (38) oberhalb des Trennbehältnisses (12) und/oder des Staudruckgefäßes (26) angeordnet ist, so dass in der Kühleinrichtung (38) entstehende Kondensflüssigkeit in die Prozessflüssigkeit fließt und zusammen mit dieser abfließt.

8. Vorrichtung nach einem der vorhergehenden Ansprüche 6 oder 7, **dadurch gekennzeichnet, dass** die Kühleinrichtung (38) eine untenliegende Zuleitung (40) für das Messgas und eine obenliegende Ableitung (42) aufweist, so dass die Kondensflüssigkeit durch die Messgaszuleitung (40) abfließen kann.

9. Vorrichtung nach einem der vorhergehenden Ansprüche 6 bis 8, **dadurch gekennzeichnet, dass** die Kühleinrichtung (38) als Pelter-Kühler ausgebildet ist.

10. Vorrichtung nach einem der vorhergehenden Ansprüche 6 bis 9, **dadurch gekennzeichnet, dass** das Messgas in der Kühleinrichtung (38) auf etwa 2°C abgekühlt wird.

## Claims

1. A device for the separation of volatile organic carbon compounds from a carrier liquid, particularly water, comprising a separating tank (12) with a carrier liquid inlet line (14), a measuring gas inlet (18), a carrier liquid drain (16) and a measuring gas flue (20), wherein in said carrier liquid drain there is provided a dynamic pressure system (26) such that the pressure in the measuring gas flue (20) can be maintained at a constant value, **characterized in that** said device is adapted such that the gas phase in the measuring gas flue (20) has an excess pressure which is maintained at a constant value by said dynamic pressure system.

2. A device as defined in claim 1, **characterized in that** said dynamic pressure system is embodied as a back pressure vessel (26).

3. A device as defined in any one of the previous claims, **characterized in that** the dynamic pressure is approximately 4 kPa.

4. A device as defined in any one of the previous claims, **characterized in that** in the carrier liquid inlet line (14) there is provided a preheating unit (22) for the carrier liquid.

5. A device as defined in any one of the previous claims, **characterized in that** the measuring gas is air.

6. A device as defined in any one of the previous claims, **characterized in that** in said measuring gas flue (20) there are disposed cooling means (38) for cooling the measuring gas.

7. A device as defined in claim 6, **characterized in that** said cooling means (38) are disposed above said separating tank (12) and/or said back pressure vessel (26) so that condensate formed in said cooling means (38) flows into said carrier liquid and drains away together with the latter.

8. A device as defined in any one of the previous claims 6 or 7, **characterized in that** said cooling means (38) exhibit a bottom feed pipe (40) for the measuring gas and a top flue (42) so that the condensate can flow back through said measuring gas pipe (40).

9. A device as defined in any one of the previous claims 6 to 8, **characterized in that** said cooling means (38) are embodied as a Peltier cooler.

10. A device as defined in any one of the previous claims 6 to 9, **characterized in that** the measuring gas is cooled in said cooling means (38) to approximately 2°C.

## Revendications

1. Dispositif pour séparer des carbures organiques volatiles d'un liquide de processus, en particulier d'eau, avec un récipient de séparation (12) avec arrivée de liquide de processus (14), arrivée de gaz de mesure (18), écoulement de liquide de processus (16) et évacuation du gaz de mesure (20), un dispositif de pression de retenue (26) étant prévu dans le circuit de liquide de processus, de sorte qu'une pression constante peut être maintenue dans l'évacuation du gaz de mesure (20), **caractérisé en ce que** le dispositif est aménagé de telle sorte que la phase gazeuse dans l'évacuation du gaz de mesure (20) a une surpression qui est maintenue constante par le dispositif de pression de retenue.

2. Dispositif selon la revendication 1, **caractérisé en ce que** le dispositif de pression de retenue est conçu comme un récipient de pression de retenue (26).

3. Dispositif selon l'une quelconque des revendications précédentes, **caractérisé en ce que** la pression de retenue est d'environ 4 kPa.

4. Dispositif selon l'une quelconque des revendications précédentes, **caractérisé en ce qu'**une unité de préchauffage (22) est prévue pour le liquide de processus dans l'arrivée de liquide de processus (14).

5. Dispositif selon l'une quelconque des revendications précédentes, **caractérisé en ce que** le gaz de mesure est de l'air.

6. Dispositif selon l'une quelconque des revendications précédentes, **caractérisé en ce qu'**un dispositif de refroidissement (38) est disposé dans l'évacuation du gaz de mesure (20) pour le refroidissement du gaz de mesure.

7. Dispositif selon la revendication 6, **caractérisé en ce que** le dispositif de refroidissement (38) est disposé au-dessus du récipient de séparation (12) et/ou du récipient de pression de retenue (26), de sorte que du liquide de condensation se formant dans le dispositif de refroidissement (38) circule dans le liquide de processus et s'évacue conjointement avec celui-ci.

8. Dispositif selon l'une quelconque des revendications précédentes 6 ou 7, **caractérisé en ce que** le dispositif de refroidissement (38) présente une arrivée (40) sous-jacente pour le gaz de mesure et une dérivation (42) sus-jacente, de sorte que le liquide de condensation peut s'écouler à travers l'arrivée du gaz de mesure (40).

9. Dispositif selon l'une quelconque des revendications précédentes 6 à 8, **caractérisé en ce que** le dispositif de refroidissement (38) est conçu comme un refroidisseur de Peltier.

10. Dispositif selon l'une quelconque des revendications 6 à 9 précédentes, **caractérisé en ce que** le gaz de mesure est refroidi jusqu'à environ 2°C dans le dispositif de refroidissement (38).
